# EUROPEAN PATENT SPECIFICATION

(11) **EP 2 462 889 B1**
(45) Date of publication and mention of the grant of the patent: **21.08.2013**
(21) Application number: 10194787.7
(22) Date of filing: 13.12.2010
(51) Int. Cl.: A61B 17/70

(54) **Bone anchoring device**
Knochenverankerungsvorrichtung
Dispositif d'ancrage d'os

(43) Date of publication of application: 13.06.2012
(73) Proprietor: Biedermann Technologies GmbH & Co. KG, 78166 Donaueschingen (DE)
(72) Inventor: Biedermann, Lutz, 78048, VS-Villingen (DE); Matthis, Wilfried, 79367, Weisweil (DE); Hägle, Tobias, 78166, Donaueschingen (DE)
(74) Representative: Prüfer & Partner GbR European Patent Attorneys

(56) References cited:
- US-A1- 2005 154 391
- US-A1- 2007 118 123
- US-A1- 2007 270 842
- US-A1- 2008 177 260
- US-A1- 2010 145 394
- US-A1- 2010 152 787
- US-A1- 2010 204 735
- US-A1- 2010 298 891

## Description

The invention relates to a bone anchoring device, which comprises a bone anchoring element for anchoring in a bone or a vertebra, a receiving part for coupling the bone anchoring element to a stabilization element such as a spinal rod, wherein the bone anchoring element is pivotable in the receiving part and can be pivoted to at least one side with an enlarged pivot angle.

A bone anchoring device of this type is described in US 6,736,820. The bone anchoring device comprises a bone screw and a receiving part with an open first bore and a substantially U-shaped cross-section for receiving the rod and a second bore on the end opposite to the first bore and a seat for the head. In order that the screw member can be pivoted to at least one side by an enlarged angle, the edge bounding the free end of the second bore is of asymmetric construction. In a modified embodiment an insert piece is provided, which has a spherical bottom as the seat for the head. This allows to change the orientation of the enlarged pivot angle.

US 2005/0154391 A1 describes a bone anchor assembly with a bone anchor and a receiving member. The receiving member has a first section having a first bore defining a first bore axis and a second section having a second bore defining a second bore axis and being sized to receive at least a portion of the bone anchor, wherein the second bore axis intersects the first bore axis. The second section is rotatable about the first bore axis. In an embodiment, the second section is seated internally within the first section.

Since the second section is rotatable with respect to the first section, it is possible to pivot bone anchors out of alignment with one another to avoid interference due to the close proximity of adjacent vertebrae.

Another polyaxial bone anchor is described in US 2007/0118123 A1, wherein a locking element is provided, which is shaped and configured to allow an anchoring member such as a screw or a hook to polyaxially rotate at large angles about a central axis of the bone anchor before compression locking the anchoring member within an anchor head.

With the bone anchoring devices mentioned before, it is difficult to adjust the position in which the screw has an enlarged pivot angle once the screw member is partly or fully screwed into the bone.

US 2008/0177260 A1 discloses a bone fastener assembly useful in constructs of stabilizing bones of a patient. In particular, the bone fastener assembly includes a rod holder, a fastener seat mounted to the rod holder for rotation about the rod holder axis and a bone fastener. The bone fastener engages the fastener seat in pivoting relationship such that the bone fastener is able to pivot to vary the angle between the bone fastener and the fastener seat.

It is the object of the invention to provide an improved bone anchoring device that facilitates the adjustment of the direction of an enlarged pivot angle of the bone anchoring element.

The object is solved by a bone anchoring device according to claim 1. Further developments are given in the dependent claims.

The bone anchoring device allows to rotate the seat member that holds the head of the screw by means of a driver inserted from the top end of the receiving part so that the direction of pivoting can be adjusted to any desired direction. The orientation of the driver, for example, the orientation of a handle of the driver, can be used as a precise indication of the orientation of the seat member. Hence, it is possible to adjust the direction of an enlarged pivot angle during surgery.

It is further possible to pre-adjust the orientation of the seat member using a single support block that fixes the seat member and then rotating the receiving part relative to the seat member.

The locking of the head in the seat member is effected by pressure exerted from above onto the head, which is then pressed against the seat. Therefore, the locking of the head is effected in a safe and predictable manner.

Further features and advantages of the invention will become apparent from the description of embodiments by means of the accompanying drawings.

In the drawings:
- Fig. 1:: shows a perspective exploded view of the bone anchoring device with a spinal rod according to an embodiment.
- Fig. 2:: shows the bone anchoring device of Fig. 1 in an assembled state without rod and fixation screw.
- Fig. 3:: shows a sectional view of the bone anchoring device of Fig. 1, the section being taken perpendicular to the rod axis.
- Fig. 4:: shows a sectional view of the bone anchoring device of Fig. 1, the section being taken along the rod axis.
- Fig. 5:: shows a perspective view of a seat member of the bone anchoring device.
- Fig. 6:: shows a side view of the seat member of Fig. 5.
- Fig. 7:: shows a sectional view of the seat member, the section being taken along line A-A in Fig. 6.
- Fig. 8:: shows a perspective view of a first pressure member of the bone anchoring device.
- Fig. 9:: shows a top view of the first pressure member of Fig. 8.
- Fig. 10:: shows a side view of the first pressure member of Fig. 8.
- Fig. 11:: shows a sectional view of the first pressure member along line B-B of Fig. 10.
- Fig. 12:: shows a perspective view of a second pressure member of the bone anchoring device from below.
- Fig. 13:: shows a top view of the second pressure member of Fig. 12.
- Fig. 14:: shows a sectional view of the second pressure member along line C-C of Fig. 13.
- Fig. 15:: shows a sectional view of the bone anchoring element, the seat member and the first pressure member.
- Fig. 16:: shows a perspective view of an assembly including the bone anchoring element, the seat member and the first pressure member.
- Figs. 17a): to 17c): shows steps of use of the bone anchoring device according to the first embodiment.
- Figs. 18a): to 18c): show steps adjusting the seat member relative to the receiving part according to a first example of a method.
- Fig. 19:: shows a top view of a support block for adjusting the seat member relative to the receiving part.
- Fig. 20:: shows a sectional view of the support block with the bone anchoring device inserted, the section being taken along the rod axis.
- Fig. 21: shows a perspective view of the bone anchoring device with an insertion tool.
- Fig. 22: shows an enlarged portion of the the device of Fig. 21.
- Fig. 23: shows a perspective view of the bone anchoring device and a portion of another example for an insertion tool.

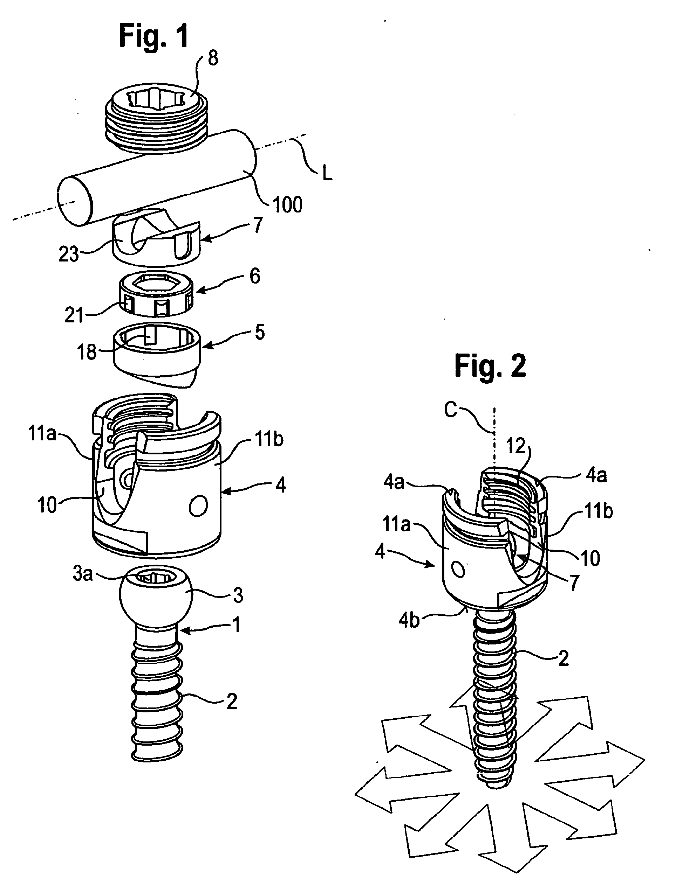

As shown in Figs. 1 and 2, a bone anchoring device according to an embodiment comprises a bone anchoring element 1 in the form of a bone screw having a threaded shaft 2 and a head 3. The head 3 is typically spherically-shaped and has a recess 3 a for engagement with a driver. The head 3 is held in a receiving part 4 that couples the bone anchoring element 1 to a stabilization rod 100. In the receiving part 4, a seat member 5 providing a seat for the head 3 of the bone anchoring element 1, a first pressure member 6 and a second pressure member 7 for exerting pressure onto the head 3 of the bone anchoring element are arranged. Further, a fixation element in the form of a fixation screw 8 is provided for securing and fixing the rod 100 in the receiving part 4. When the bone anchoring device is assembled but the angular position of the bone anchoring element not yet fixed, the bone anchoring element 1 can pivot with an enlarged pivot angle in an unlimited number of directions as shown by the arrows in Fig. 2, depending on the position of the seat member 5 within the receiving part 4.

The receiving part 4 will be described with reference to Figs. 1 to 4. It has a top end 4a and a bottom end 4b, a central axis C and a coaxial bore 9 extending from the top end 4a to the bottom end 4b. Adjacent to the top end 4a a substantially U-shaped recess 10 is provided, which forms a channel for receiving the rod 100. By means of the recess 10 two free legs 11a, 11b are formed, which are provided with an internal thread 12 cooperating with the fixation screw 8. At the lower portion of the bore 9 near the bottom end 4b a stop 13 in the form of an annular edge is provided for preventing the seat member 5 from escaping through the opening 14 at the bottom end 4b. It shall be noted that the bore 9 is not restricted to the specific shape of the bore showing in the drawings but can have different portions with different diameters.

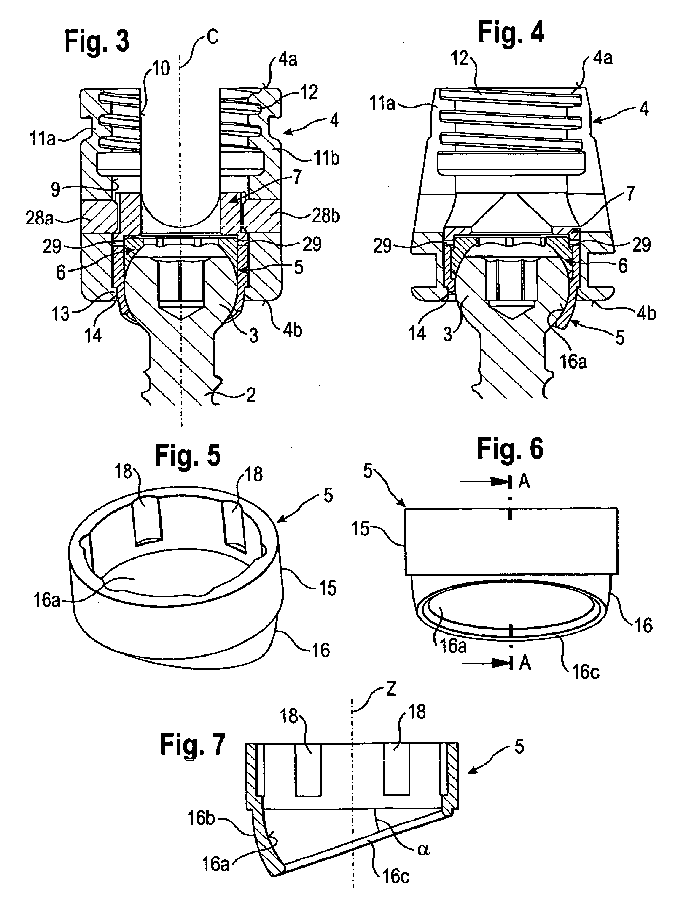

The seat member 5 is shown in particular in Figs. 5 to 7. It comprises a hollow cylindrical first portion 15, the inner diameter of which is slightly larger than the maximum diameter of the spherically-shaped head 3 and the outer diameter, of which is slightly smaller than the inner diameter of the bore 9, so that the seat member 5 insertable into the bore 9. The axial length of the first portion 15 is such that when the head 3 rests within the seat portion 16, the first portion 15 projects above the head 3. Adjacent the first portion 15, the seat member 5 comprises a hollow second portion 16, which has an internal spherically-shaped surface 16a for providing a seat for the spherically-shaped head 3. The outer surface 16b of the second portion 16 is also spherically-shaped with an outer diameter that is smaller than the outer diameter of the first hollow cylindrical portion 15.

As can be seen in particular in Fig. 7, the second portion 16 is asymmetric with respect to a plane including the cylinder axis Z. This is achieved in that the lower edge 16c is inclined with respect to the hollow cylindrical first portion 15 at an angle ∝. By means of this, the screw element 1 can pivot when it is seated in the seat 16a at a greater angle to one side relative to the cylinder axis Z compared to the opposite side. It shall be noted that the member 5 can be sized such that the pivot angle of the screw element with respect to the one side can be only as much as approximately 10° and can be as much as approximately 40° to 50° to the opposite side. At the same time, the size of the pivot angle is limited to the extent that the head has still an adequate support in the seat member. The second portion 16 is not limited to the design shown. For example, the asymmetry can be generated by a cut-out in a hollow spherical-shaped portion allowing the shank to pivot only through the cut-out. The inner surface 16a is shown as being a spherically-shaped surface. However, it can have another shape, such as, for example any tapering shape, which is sized so as to prevent falling out of the head 3.

The seat member 5 further comprises a plurality of coaxial recesses 18 on the inner wall of the first portion 15, which open to the free end of the first portion 15 and end at a distance from the lower end of the first portion 15. The recesses 18 are provided for a form locking engagement with corresponding projections on the first pressure element 6. The number and shape of the recesses 18 is not limited to the number and shape of the recesses shown in the Figures, for example, only one recess is necessary for a form lock connection between the first pressure element 6 and the seat member 5.

As can be seen in particular in Figs. 3 and 4, the seat member 5 abuts on the annular edge 13 of the receiving part, when it is inserted into the receiving part 4 and moved downward along the bore 9. At least a portion of the second portion 16 of the seat member extends out of the lower opening 14 of the receiving part. The seat member 5 is rotatable within the receiving part 4, so that the inclined edge 16c can assume any position relative to the U-shaped recess 10. As a consequence thereof, the position with the enlarged pivot angle of the anchoring element 1 can assume any orientation with respect to the U-shaped recess 10 and therefore with respect to the rod axis L.

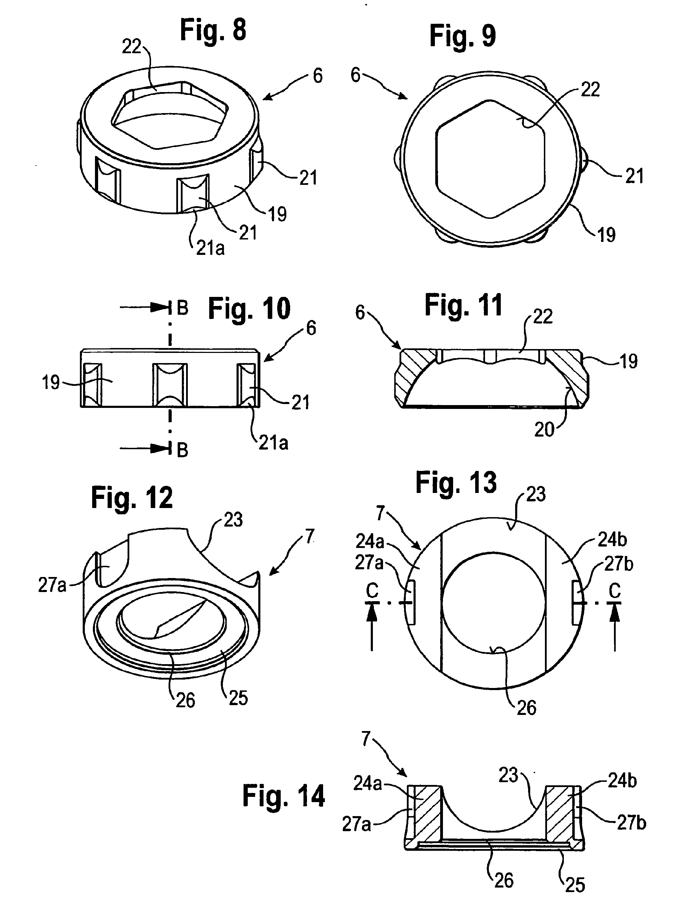

The first pressure member 6 will now be explained specifically with reference to Figs. 8 to 11. The first pressure member 6 has a substantially hollow cylindrical portion 19, the outer diameter of which is only slightly smaller than the inner diameter of the first hollow cylindrical portion 15 of the seat member so that, as can be seen in Figs. 3 and 4, the first pressure member 6 can be inserted into the first portion 15 of the seat member 5 and is axially movable with respect to the seat member. On its side facing the head 3, the first pressure member 6 has a recess 20, which is in this embodiment a spherically-shaped recess with an inner diameter adapted to the diameter of the head 3, so that the first pressure member 6 can exert pressure onto the head 3. When the first pressure member 6 is inserted, it rests on the head 3. It has such an axial length that it projects above the seat member 5, as can be seen in Figs. 3 and 4. At the outer surface of the cylindrical portion 19 a plurality of coaxially extending projections 21 are provided, which engage the recesses 18 of the seat member 5 to form a form locking connection between the first pressure member and the seat member. The recesses 18 at the seat member element form also guides for an axial movement of the first pressure member. Instead of the plurality of recesses and projections at the inner surface of the seat member and the outer surface of the first pressure member, respectively, any other connection between the seat member and the first pressure member, which allows the transmission of rotational forces from the first pressure member to the seat member and is conceivable. The projections 21 end at a distance from the top end and can comprise a chamfer 21a to facilitate engagement with the corresponding recesses 18 of the seat member 5.

On its end opposite to the recess 20 the first pressure element comprises an engagement structure 22 for engagement with a driver, in the embodiment shown, it is a hexagon recess.

The second pressure member 7 will be explained with reference to Figs. 12 to 14. The second pressure element 7 is substantially cylindrical and has on its side opposite to the head 3 a cylindrically-shaped recess 23, which is sized so as to guide the rod 100 therein. By means of the recess 23 two legs 24a, 24b are formed. On its side opposite to the recess 23, the second pressure member comprises a coaxial recess 25, which is sized such that the second pressure member can rest on the cylindrical portion 19 of the first pressure member. The second pressure member 7 further comprises a shallow coaxial bore 26 to allow access to the first pressure member with a driver. On the outer sidewall of the legs 24a, 24b two coaxial recesses 27a, 27b are formed, which are open to the top side and close to the bottom side by means of which the second pressure member 7 can be prevented from rotating and from escaping through the top end 4a of the receiving part 4, for example, by means of pins 28a, 28b provided at the receiving part as shown in Fig. 3.

When the second pressure element is inserted, it abuts on the first pressure element in such a way that there is a gap 29 between the seat member 5 and the second pressure element 7, so that the second pressure element 7 does not press onto the seat member 5.

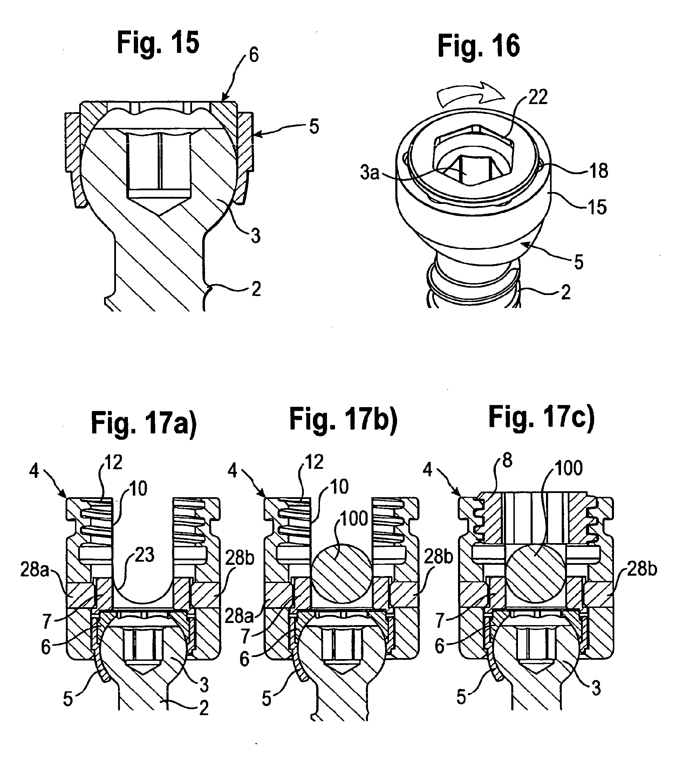

As shown in Figs. 15 and 16, the form lock connection between the first pressure element 6 and the seat member 5 allows to transmit a rotational force exerted by driver, which engages the first pressure element at the recess 22, to the seat member 5, so that the seat member 5 rotates with the first pressure element 6. Since the form lock connection between the first pressure member 6 and the seat member 5 is such that the first pressure member can slide in an axial direction with respect to the seat member, the second pressure element can exert pressure onto the first pressure element to press the head 3 against the seat member 5.

The bone anchoring device as a whole or in part is made of a bio-compatible material, such as a bio-compatible metal, such as titanium, stainless steel, bio-compatible alloys such as nitinol or others or bio-compatible plastic materials, such as, for example, medical grade polyethere-therketone (PEEK). The parts of the bone anchoring device can be made all of the same material or can be made of different materials.

The steps of use of the bone anchoring device are shown in Figs. 17a)-17c). The bone anchoring device may be pre-assembled in such a way that the seat member 5, the bone anchoring element 1, the first pressure element 6 and the second pressure element 7 are inserted into the receiving part 4 and secured against falling out as well as against rotation of the second pressure element 7, for example, by means of the pins 28a, 28b. In this condition, the cylindrically-shaped recess 23 of the second pressure element is aligned with the U-shaped recess 12 of the receiving part. The seat member abuts on the annular edge 13 of the receiving part. In the pre-assembled state, the head 3 can freely pivot within the seat member 5. In a next step, the bone anchoring element 1 is screwed into a bone part or a vertebra. Usually a plurality of bone anchoring elements are used and connected through the rod 100. Before insertion of the rod 100, the receiving parts are aligned through pivoting the receiving parts relative to the head 3. To adjust the position of the seat member 5 in view of the desired direction with enlarged pivot angle, the first pressure element 6 is rotated with a driver. Since the seat member 5 is positively connected to the first pressure member 6, it rotates with the first pressure member 6. Then, as shown in Fig. 17b), the rod is inserted. Next, as shown in Fig. 17c) the fixation screw 8 is screwed-in between the legs of the receiving part and tightened so that the rod 100 presses onto the second pressure element 7, which itself presses onto the first pressure element thereby locking the head in the seat member 5. Simultaneously, the seat member is pressed against the annular edge 13 and is thereby fixed in its rotational position.

Modifications of the embodiment are conceivable. For example, it is possible to omit the second pressure element and to press directly with the rod onto the first pressure element. In a further modification, the second pressure element has legs, which extend above the rod and the fixation element 8 is a two-part fixation element with an outer screw acting onto the second pressure element and an inner screw acting onto the rod. In a further modification, all kinds of bone anchors can be used for the bone anchoring element, such as bone nails, other types of bone screws, canulated screws, etc. The head and the shaft of the bone anchoring element may be separate parts that are connectable to each other.

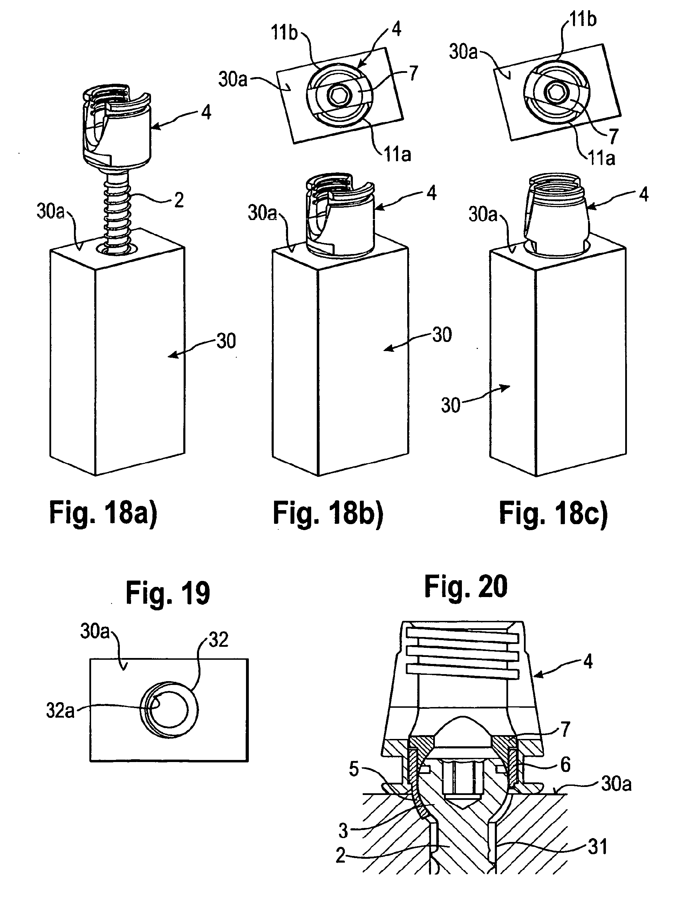

Figs. 18 to 20 show an example of a tool and a method for adjusting the rotational position of the seat member 5. A support block 30 is provided, which comprises a longitudinal bore 31 extending from a top surface 30a into the support block 30. In the top surface 30a a recess 32 is formed that extends into the support block, which has an inner surface 32a corresponding to the outer surface 16b of the second portion 16 of the seat member 5. Hence, the recess 32 is asymmetrical and adapted to the shape of the lower portion 16 of the seat member 5. As shown in Figs. 19 and 20, when the pre-assembled bone anchoring device is inserted with the threaded shaft 2 into the hole 31, the second portion 16 of the seat member rests in the recess 32 and is prevented from rotating therein due to the form lock connection.

Once the bone anchoring device is fully inserted in the bore 31, the screw element 1 and the receiving part including the second pressure element 7 are rotatable with respect to the seat member 5 and the first pressure element 6. Hence, the receiving part can be rotated with respect to the seat member and the first pressure element, so that the seat member 5 has the desired orientation. Two examples are shown in Figs. 18b and 18c.

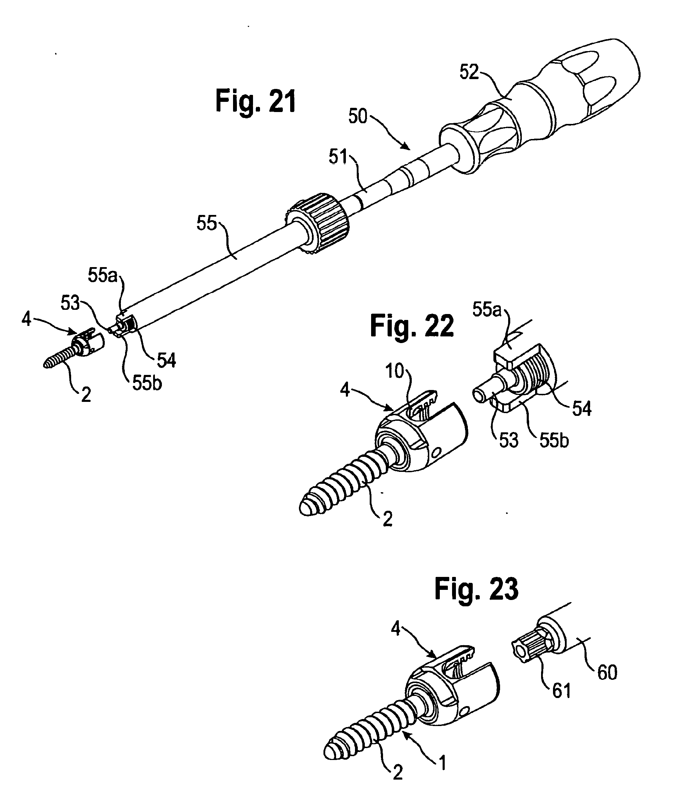

The adjustment using the support block can be made before the actual surgery takes place and can serve for a pre-orientation of the seat member 5. For holding this orientation of the seat member an insertion tool for inserting the screw member into the bone can be used which is shown in Figs. 21 and 22. The insertion tool 50 has a first portion 51 with a handle 52 at one end, a tip 53 at the other end and adjacent the tip an externally threaded portion 54 cooperating with the internal thread 12 on the legs of the receiving part 4. The tool further has a second portion 55 with two side flanges 55a, 55b engaging the U-shaped recess 10 of the receiving part. The first portion 51 is rotatable with respect to the second portion 55. When the tool engages the bone anchoring device, the second portion 55 holds the receiving part through engagement with the U-shaped recess and the tip 53 presses onto the screw head when the threaded portion 54 is screwed between the legs. The orientation of the seat member is thereby secured by the pre-tension and the shaft can be inserted into the bone with the insertion tool.

In an alternative method, the bone anchoring device can be inserted into the bone with a tool 60 shown in Fig. 23 which has a simple drive 61 portion at its end, for example a torx drive. In this case, the rotational orientation of the seat member is not maintained during insertion. After insertion of the shaft, a separate driver for engagement with the recess 22 in the first pressure element is used to adjust the rotational position of the seat member from the top end. A visible mark can be provided at the drive to serve as an indication for the position of the seat member.

## Claims

1. Bone anchoring device comprising
a bone anchoring element (1) having a head (3) and a shaft (3) configured to be anchored in a bone or a vertebra;
a receiving part (4) for coupling the bone anchoring element to a stabilization element (100), the receiving part having
a top end (4a) and bottom end (4b), a bore (9) extending from the top end to the bottom end and having a bore axis (C),
a recess (10) for receiving the stabilization element, the recess being in communication with said bore (9);
a seat member (5) arranged within the bore (9) and being rotatable around the bore axis, the seat member forming a seat for pivotably holding the head, wherein the seat member has an inclined lower edge (16c) so as to provide an enlarged pivot angle of the bone anchoring element to one side compared to the other side relative to the bore axis (C);
a pressure member (6) which contacts the head to exert pressure onto the head, wherein the pressure member (6) is connected to the seat member (5) so as to transmit rotational forces to the seat member (5) in order to allow an angle adjustment of the seat member about the bore axis (C).

2. The bone anchoring device of claim 1 wherein the pressure member (6) is axially movable with respect to the seat member (5).

3. The bone anchoring device of one of claims 1 to 2, wherein the seat member (5) abuts against a stop (13) in the receiving part (4).

4. The bone anchoring device of one of claims 1 to 3, wherein the seat member (5) has an at least partially spherical inner surface (16a).

5. The bone anchoring device of one of claims 1 to 4, wherein the seat member (5) projects at least partly out of the receiving part (4) at the bottom end (4b).

6. The bone anchoring device of one of claims 1 to 5, wherein the pressure member (6) and the seat member (5) are connected via a form lock connection.

7. The bone anchoring device of claim 5, wherein the seat member (5) comprises at least one axial recess (18) or projection which engages with a corresponding projection (21) or recess at the pressure member 86).

8. The bone anchoring device of one of claims 1 to 7, wherein the pressure member has an engagement portion (22) for engagement with a driver.

9. The bone anchoring device of claim 8, wherein the engagement portion is provided at a side of the pressure member which faces away from the head (3).

10. The bone anchoring device of one of claims 1 to 9, wherein the seat member (5) has a cylindrical portion (15) and the pressure member (6) has a cylindrical portion which are axially movable relative to each other but rotationally fixed.

11. The bone anchoring device of one of claims 1 to 10, wherein the pressure member (6) is a first pressure member and wherein a second pressure member (7) is provided in the receiving part on top of the first pressure member and wherein the second pressure member (7) has a recess (23) for guiding the fixation element (100).

12. The bone anchoring device of claim 11, wherein in a state in which the head is locked in the seat member (5) the second pressure member presses onto the first pressure member (6) without touching the seat member.

13. The bone anchoring device of claim 11 or 12, wherein the second pressure member is rotatable with respect to the first pressure member (6).

14. The bone anchoring device of one of claims 1 to 13, wherein the second pressure member (7) is secured against rotation in the receiving part (4).

15. The bone anchoring device of one of claims 1 to 14, wherein the recess (10) for the rod is U-shaped.

16. A method for adjusting the pivot angle of a polyaxial bone anchoring device, wherein the bone anchoring device comprises
a bone anchoring element (1) having a head (3) and a shaft (3) configured to be anchored in a bone or a vertebra;
a receiving part (4) for coupling the bone anchoring element to a stabilization element (100), the receiving part having
a top end (4a) and bottom end (4b), a bore (9) extending from the top end to the bottom end and having a bore axis (C),
a substantially U-shaped recess (10) for receiving the stabilization element, the recess being in communication with said bore (9);
a seat member (5) arranged within the bore (9) and being rotatable around the bore axis, the seat member forming a seat for pivotably holding the head, wherein the seat member has an inclined lower edge (16c) so as to provide an enlarged pivot angle of the bone anchoring element to one side compared to the other side relative to the bore axis (C);
a pressure member (6) which contacts the head to exert pressure onto the head, wherein the pressure member (6) is connected to the seat member (5) so as to transmit rotational forces to the seat member (5) in order to allow an angle adjustment of the seat member about the bore axis (C);
wherein the seat member (5) is supported in a recess (32a) in the support device (30) in a form locking manner;
the method comprising the steps:
supporting the seat member or the receiving part (4) in a support device (30);
rotating the receiving part (4) with respect to the seat member (5) to adjust the orientation of an enlarged pivot angle.

## Patentansprüche

1. Knochenverankerungsvorrichtung, umfassend:
ein Knochenverankerungselement (1) mit einem Kopf (3) und einem Schaft (2), das eingerichtet ist, in einem Knochen oder einem Wirbel verankert zu werden;
ein Aufnahmeteil (4) zum Koppeln des Verankerungselements an ein Stabilisierungselement (100), wobei das Aufnahmeteil aufweist:
ein oberes Ende (4a) und ein unteres Ende (4b), sowie eine Bohrung (9), die sich von dem oberen Ende zu dem unteren Ende erstreckt und eine Bohrungsachse (C) besitzt,
eine Ausnehmung (10) zum Aufnehmen des Stabilisierungselements, wobei die Ausnehmung in Verbindung mit der Bohrung (9) steht;
ein Sitzelement (5), das innerhalb der Bohrung (9) und drehbar um die Bohrungsachse angeordnet ist, wobei das Sitzelement einen Sitz für das schwenkbare Halten des Kopfs ausbildet, wobei das Sitzelement eine geneigte untere Kante (16c) aufweist, so dass ein vergrößerter Schwenkwinkel des Knochenverankerungselements auf der einen Seite im Vergleich zu der anderen Seite relativ zu der Bohrungsachse (C) bereitgestellt wird;
ein Druckelement (6), welches den Kopf kontaktiert, um einen Druck auf den Kopf auszuüben, wobei das Druckelement (6) mit dem Sitzelement (5) verbunden ist, um Drehkräfte auf das Sitzelement (5) zu übertragen, um eine Winkeleinstellung des Sitzelements um die Bohrungsachse (C) herum zu ermöglichen.

2. Die Knochenverankerungsvorrichtung gemäß Anspruch 1, wobei das Druckelement (6) in Bezug auf das Sitzelement (5) axial bewegbar ist.

3. Die Knochenverankerungsvorrichtung gemäß einem der Ansprüche 1 bis 2, wobei das Sitzelement (5) gegen einen Anschlag (13) in dem Aufnahmeteil (4) anstößt.

4. Die Knochenverankerungsvorrichtung gemäß einem der Ansprüche 1 bis 3, wobei das Sitzelement (5) eine zumindest teilweise sphärische innere Oberfläche (16a) besitzt.

5. Die Knochenverankerungsvorrichtung gemäß einem der Ansprüche 1 bis 4, wobei das Sitzelement (5) zumindest teilweise aus dem Aufnahmeteil (4) an dessen unterem Ende (4b) hervorsteht.

6. Die Knochenverankerungsvorrichtung gemäß einem der Ansprüche 1 bis 5, wobei das Druckelement (6) und das Sitzelement (5) miteinander über eine formschlüssig sperrende Verbindung miteinander verbunden sind.

7. Die Knochenverankerungsvorrichtung gemäß Anspruch 5, wobei das Sitzelement (5) zumindest eine axiale Ausnehmung (18) oder einen Vorsprung auffasst, welche beziehungsweise welcher entsprechend mit einem Vorsprung (21) oder einer Ausnehmung an dem Druckstück (6) in Eingriff tritt.

8. Die Knochenverankerungsvorrichtung gemäß einem der Ansprüche 1 bis 7, wobei das Druckelement einen Eingriffsabschnitt (22) zum Eingreifen anhand eines Werkzeugs aufweist.

9. Die Knochenverankerungsvorrichtung gemäß Anspruch 8, wobei der Eingriffsabschnitt auf einer Seite des Druckelements vorgesehen ist, welche von dem Kopf (3) abgewandt ist.

10. Die Knochenverankerungsvorrichtung gemäß einem der Ansprüche 1 bis 9, wobei das Sitzelement (5) einen zylindrischen Abschnitt (15) und das Druckelement (6) einen zylindrischen Abschnitt besitzt, welche relativ zueinander in axialer Richtung bewegbar, aber in Drehrichtung fixiert sind.

11. Die Knochenverankerungsvorrichtung gemäß einem der Ansprüche 1 bis 10, wobei das Druckelement (6) ein erstes Druckelement ist, und wobei ein zweites Druckelement (7) in dem Aufnahmeteil auf dem ersten Druckelement vorgesehen ist, und wobei das zweite Druckelement (7) eine Ausnehmung (23) zum Führen des Fixationselements (100) besitzt.

12. Die Knochenverankerungsvorrichtung gemäß Anspruch 11, wobei in einem Zustand, in welchem der Kopf in dem Sitzelement (5) gesperrt ist, das zweite Druckelement auf das erste Druckelement (6) drückt, ohne das Sitzelement zu berühren.

13. Die Knochenverankerungsvorrichtung gemäß Anspruch 11 oder 12, wobei das zweite Druckelement drehbar in Bezug auf das erste Druckelement (6) ist.

14. Die Knochenverankerungsvorrichtung gemäß einem der Ansprüche 1 bis 13, wobei das zweite Druckelement (7) gegen Drehung in dem Aufnahmeteil (4) gesichert ist.

15. Die Knochenverankerungsvorrichtung gemäß einem der Ansprüche 1 bis 14, wobei die Ausnehmung (10) für den Stab U-förmig ist.

16. Ein Verfahren zum Einstellen des Schwenkwinkels einer polyaxialen Knochenverankerungsvorrichtung, wobei die Knochenverankerungsvorrichtung umfasst:
ein Knochenverankerungselement (1) mit einem Kopf (3) und einem Schaft (2), das eingerichtet ist, in einem Knochen oder einem Wirbel verankert zu werden;
ein Aufnahmeteil (4) zum Koppeln des Knochenverankerungselements an ein Stabilisierungselement (100), wobei das Aufnahmeteil besitzt:
ein oberes Ende (4a) und ein unteres Ende (4b), und eine Bohrung (9), die sich von dem oberen Ende zu dem unteren Ende erstreckt und eine Bohrungsachse (C) besitzt,
eine im Wesentlichen U-förmige Ausnehmung (10) zum Aufnehmen des Stabilisierungselements, wobei die Ausnehmung in Verbindung mit der Bohrung (9) steht;
ein Sitzelement (5), das innerhalb der Bohrung (9) und um die Bohrungsachse herum drehbar angeordnet ist, wobei das Sitzelement einen Sitz zum schwenkbaren Halten des Kopfs ausbildet, wobei das Sitzelement eine geneigte untere Kante (16c) besitzt, um einen vergrößerten Schwenkwinkel des Knochenverankerungselements auf der einen Seite im Vergleich zu der anderen Seite relativ zu der-Bohrungsachse (C) bereitzustellen;
ein Druckelement (6), welches den Kopf kontaktiert, um Druck auf den Kopf auszuüben, wobei das Druckelement (6) mit dem Sitzelement (5) verbunden ist, um Drehkräfte auf das Sitzelement (5) zu übertragen, um eine Winkeleinstellung des Sitzelements um die Bohrungsachse (C) herum zu ermöglichen;
wobei das Sitzelement (5) in einer Ausdehnung (32a) in der Haltevorrichtung (30) in der Art formschlüssiger Sperrung gehalten ist;
wobei das Verfahren die Schritte umfasst:
Halten des Sitzelements oder des Aufnahmeteils (4) in einer Haltevorrichtung (30);
Drehen des Aufnahmeteiles (4) in Bezug auf das Sitzelement (5), um die Ausrichtung eines vergrößerten Schwenkwinkels einzustellen.

## Revendications

1. Dispositif d'ancrage osseux comprenant
un élément d'ancrage osseux (1) ayant une tête (3) et un arbre (3) configuré pour être ancré dans un os ou une vertèbre ;
une partie de réception (4) destinée à coupler l'élément d'ancrage osseux à un élément de stabilisation (100), la partie de réception ayant
une extrémité supérieure (4a) et une extrémité inférieure (4b), un alésage (9) s'étendant depuis l'extrémité supérieure jusqu'à l'extrémité inférieure et ayant un axe d'alésage (C),
un évidement (10) destiné à recevoir l'élément de stabilisation, l'évidement étant en communication avec ledit alésage (9) ;
un élément de siège (5) agencé dans l'alésage (9) et pouvant tourner autour de l'axe d'alésage, l'élément de siège formant un siège pour maintenir en pivotement la tête, où l'élément de siège a un bord inférieur incliné (16c) de manière à fournir un angle de pivotement élargi de l'élément d'ancrage osseux à un côté comparé à l'autre côté par rapport à l'axe d'alésage (C) ;
un élément de pression (6) qui entre en contact avec la tête pour exercer une pression sur la tête, où l'élément de pression (6) est relié à l'élément de siège (5) de manière à transmettre des forces de rotation à l'élément de siège (5) afin de permettre un réglage d'angle de l'élément de siège autour de l'axe d'alésage (C).

2. Dispositif d'ancrage osseux de la revendication 1, dans lequel l'élément de pression (6) est axialement mobile par rapport à l'élément de siège (5).

3. Dispositif d'ancrage osseux de l'une des revendications 1 à 2, dans lequel l'élément de siège (5) vient en butée contre une butée (13) dans la partie de réception (4).

4. Dispositif d'ancrage osseux de l'une des revendications 1 à 3, dans lequel l'élément de siège (5) a au moins une surface interne partiellement sphérique (16a).

5. Dispositif d'ancrage osseux de l'une des revendications 1 à 4, dans lequel l'élément de siège (5) fait saillie au moins partiellement hors de la partie de réception (4) au niveau de l'extrémité inférieure (4b).

6. Dispositif d'ancrage osseux de l'une des revendications 1 à 5, dans lequel l'élément de pression (6) et l'élément de siège (5) sont reliés par l'intermédiaire d'une liaison par complémentarité de forme.

7. Dispositif d'ancrage osseux de la revendication 5, dans lequel l'élément de siège (5) comprend au moins un évidement axial (18) ou une projection axiale qui s'engage avec une projection correspondante (21) ou un évidement correspondant au niveau de l'élément de pression (86).

8. Dispositif d'ancrage osseux de l'une des revendications 1 à 7, dans lequel l'élément de pression a une partie d'engagement (22) permettant un engagement avec un dispositif d'entraînement.

9. Dispositif d'ancrage osseux de la revendication 8, dans lequel la partie d'engagement est prévue au niveau d'un côté de l'élément de pression qui est opposé à la tête (3).

10. Dispositif d'ancrage osseux de l'une des revendications 1 à 9, dans lequel l'élément de siège (5) a une partie cylindrique (15) et l'élément de pression (6) a une partie cylindrique qui sont mobiles axialement l'une par rapport à l'autre, mais fixées en rotation.

11. Dispositif d'ancrage osseux de l'une des revendications 1 à 10, dans lequel l'élément de pression (6) est un premier élément de pression, et où un deuxième élément de pression (7) est prévu dans la partie de réception sur la partie supérieure du premier élément de pression et où le deuxième élément de pression (7) a un évidement (23) pour guider l'élément de fixation (100).

12. Dispositif d'ancrage osseux de la revendication 11, dans lequel dans un état dans lequel la tête est bloquée dans l'élément de siège (5), le deuxième élément de pression appuie sur le premier élément de pression (6) sans toucher l'élément de siège.

13. Dispositif d'ancrage osseux de la revendication 11 ou 12, dans lequel le deuxième élément de pression peut tourner par rapport au premier élément de pression (6).

14. Dispositif d'ancrage osseux de l'une des revendications 1 à 13, dans lequel le deuxième élément de pression (7) est fixé pour éviter une rotation dans la partie de réception (4).

15. Dispositif d'ancrage osseux de l'une des revendications 1 à 14, dans lequel l'évidement (10) pour la tige est en forme de U.

16. Procédé destiné à régler l'angle de pivotement d'un dispositif d'ancrage osseux polyaxial, dans lequel le dispositif d'ancrage osseux comprend
un élément d'ancrage osseux (1) ayant une tête (3) et un arbre (3) configuré pour être ancré dans un os ou une vertèbre ;
une partie de réception (4) destinée à coupler l'élément d'ancrage osseux à un élément de stabilisation (100), la partie de réception ayant
une extrémité supérieure (4a) et une extrémité inférieure (4b), un alésage (9) s'étendant depuis l'extrémité supérieure jusqu'à l'extrémité inférieure et ayant un axe d'alésage (C),
un évidement essentiellement en forme de U (10) destiné à recevoir l'élément de stabilisation, l'évidement étant en communication avec ledit alésage (9) ;
un élément de siège (5) agencé dans l'alésage (9) et pouvant tourner autour de l'axe d'alésage, l'élément de siège formant un siège pour maintenir en pivotement la tête, où l'élément de siège a un bord inférieur incliné (16c) de manière à fournir un angle de pivotement élargi de l'élément d'ancrage osseux à un côté comparé à l'autre côté par rapport à l'axe d'alésage (C) ;
un élément de pression (6) qui entre en contact avec la tête pour exercer une pression sur la tête, où l'élément de pression (6) est relié à l'élément de siège (5) de manière à transmettre des forces de rotation à l'élément de siège (5) afin de permettre un réglage d'angle de l'élément de siège autour de l'axe d'alésage (C) ;
dans lequel l'élément de siège (5) est supporté dans un évidement (32a) dans le dispositif de support (30) par complémentarité de forme ;
le procédé comprenant les étapes consistant :
à supporter l'élément de siège ou la partie de réception (4) dans un dispositif de support (30) ;
à faire tourner la pièce de réception (4) par rapport à l'élément de siège (5) pour régler l'orientation d'un angle de pivotement élargi.
